# EUROPEAN PATENT SPECIFICATION

(11) **EP 4 351 670 B1**
(45) Date of publication and mention of the grant of the patent: **12.11.2025**
(21) Application number: 22751214.2
(22) Date of filing: 11.07.2022
(51) Int. Cl.: A61L 27/44, A61L 24/00, A61L 27/52, A61L 27/54

(54) **RADIOPAQUE COMPOSITIONS**
RÖNTGENDICHTE ZUSAMMENSETZUNGEN
COMPOSITIONS RADIO-OPAQUES

(30) Priority: 12.07.2021 US 202163220841 P
(43) Date of publication of application: 17.04.2024
(73) Proprietor: Boston Scientific Scimed Inc., Maple Grove, Minnesota 55311 (US)
(72) Inventor: BURNETT, David L., Woking Aberdeenshire GU23 7EJ (GB); WILLIS, Sean L., Farnham Aberdeenshire GU1 03AR (GB); PARISI, Cristian, London Aberdeenshire TW9 4FA (GB)
(74) Representative: Vossius & Partner Patentanwälte Rechtsanwälte mbB
(86) International application number: PCT/US2022/036730
(87) International publication number: WO 2023/287727

(56) References cited:
- WO-A1-01/68720
- WO-A2-2020/003147
- US-A1- 2005 287 216

## Description

### CROSS-REFERENCE TO RELATED APPLICATIONS

This application claims the benefit of U.S. Provisional Patent Application No. 63/220,841 filed July 12, 2021 and entitled "RADIOPAQUE COMPOSITIONS".

### FIELD

The present disclosure relates to radiopaque compositions comprising polymers and spherical metallic particles and to their use, particularly in methods of medical treatment, including therapeutic embolization, and in the manufacture of medical devices.

### BACKGROUND

Spherical tantalum particles, also referred to as spherical tantalum powders or tantalum microspheres, are made by plasma inert gas atomization of normal tantalum particles such as angular tantalum powder and nodular tantalum powder. Existing applications for spherical tantalum include sputtering targets, high temperature or corrosion resistant coatings, and alloy additives. Items such as implants can be made entirely from 3D printed tantalum, which has excellent biocompatibility and supports enhanced bone ingrowth.

Therapeutic embolization is a minimally invasive procedure in which a material is introduced into a blood vessel to produce an occlusion in order to slow or stop blood flow or to fill a space such as an aneurism. This approach has been useful in the treatment of conditions such as gastrointestinal bleeding, arteriovenous malformations, hypervascular tumors, benign growths such as uterine fibroids, and benign prostate hyperplasia (BPH).

WO 01/68720 A1 discloses embolic compositions used as liquid embolic agents and formed into a hydrogel in situ. The embolic compositions comprise macromers having a backbone of a polymer having units with 1.2-diol and/or 1,3-diol structures. Such polymers include PVA and hydrolyzed copolymers of vinyl acetate. The embolic composition may additionally contain a copolymerisable monomer and can be delivered with a syringe. A water insoluble contrast agent e.g. tantalum or tungsten can be included in the embolic composition.

US 2005/287216 A1 is directed to syringe-derivable compositions containing particulate imaging agents such compositions being useful in therapeutic treatments involving endovascular access to a mammalian body and provides medically useful liquid compositions containing insoluble particulate imaging agents, wherein the individual particles of such imaging agents exhibit a regular, smooth morphology and a uniform particle size distribution so as to affect the enhanced performance of these compositions. In one embodiment, the compositions are useful for the embolization of blood vessels. Insoluble particulate imaging agents include tantalum and tungsten.

### SUMMARY

The scope of this invention is defined by the claims. Any references in the description to methods of treatment refer to the products of the present invention for use in a method for treatment.

In some embodiments, the present disclosure pertains to liquid compositions for medical use that comprise (a) a polymer, a macromonomer, or a combination of the foregoing and (b) spherical metallic particles; wherein at least a portion of the polymer comprises pendant iodine-containing groups and/or wherein at least a portion of the macromonomer comprises pendant iodine-containing groups; and wherein the spherical metallic particles are selected from tantalum, tungsten, rhenium, niobium, molybdenum, and alloys of the foregoing.

### (Spherical metallic particles may also be referred to herein as spherical metallic powders or metallic microspheres.)

In some embodiments, the spherical metallic particles have a median particle size ranging from 1 to 10 microns.

In some embodiments, which can be used in conjunction with any of the above embodiments, the spherical metallic particles have an average aspect ratio ranging from 1.0 to 1.25.

In some embodiments, which can be used in conjunction with any of the above embodiments, the spherical metallic particles have D10 and D90 values that are within 50% of the median diameter.

In some embodiments, which can be used in conjunction with any of the above embodiments, the spherical metallic particles have a median particle size ranging from 1 to 10 microns.

In some embodiments, the liquid compositions may contain from 0.1 g/ml or less to 0.9 g/ml or more of the spherical metallic particles, for example, ranging from 0.1 to g/ml to 0.2 g/ml to 0.3 g/ml to 0.4 g/ml to 0.5 g/ml to 0.6 g/ml to 0.7 g/ml to 0.8 g/ml to 0.9 g/ml (in other words, ranging between any two of the preceding values).

In some embodiments, which can be used in conjunction with any of the above embodiments, the liquid composition is a sterile, injectable liquid composition that solidifies in situ upon injection into a subject.

In some embodiments, which can be used in conjunction with any of the above embodiments, the liquid composition may be provided in a syringe barrel.

In some embodiments, which can be used in conjunction with any of the above embodiments, the liquid composition is a precipitating liquid composition that comprises the spherical metallic particles, the polymer, and a carrier solvent comprising a water miscible organic solvent in which the polymer is dissolved.

In some embodiments, which can be used in conjunction with any of the above embodiments, the liquid composition is a gelling liquid composition that comprises the spherical metallic particles, the polymer, and a carrier solvent in which the polymer is dissolved. The carrier solvent may be an aqueous solvent, or a water miscible organic solvent. In some embodiments, the polymer in the gelling liquid composition forms a gel in contact with a gelling agent. In some embodiments the gelling agent is a cross linking agent. In some of these embodiments the gelling agent is present naturally in bodily fluids. In some embodiments, the polymerizing liquid composition is part of a system that comprises an additional liquid composition that comprises the gelling agent.

In some embodiments, which can be used in conjunction with any of the above embodiments, the liquid composition is a polymerizing liquid composition that comprises the spherical metallic particles, the macromonomer, and optionally a carrier solution. In these embodiments, the macromonomer polymerize(s) upon contact with a polymerization initiator. In some of these embodiments, the polymerization initiator is present naturally in bodily fluids. In some of these embodiments, the polymerizing liquid composition is part of a system that comprises an additional liquid composition that comprises the polymerization initiator.

In some embodiments, which can be used in conjunction with any of the above embodiments, the liquid composition is a polymer composition capable of sol-gel phase transition in response to *in vivo* conditions including temperature, ionic strength and/or pH. This composition comprises the spherical metallic particles, the polymer capable of sol-gel phase transition and optionally a carrier solution. In these embodiments, the polymer is typically a block co-polymer comprising at least an A block and a B block. Examples of such polymers are described in US6562362, US6660247, US6784266, US7485317 and US7025990.

In some embodiments, which can be used in conjunction with any of the above embodiments, the polymer is a hydrophilic polymer or a hydrophobic polymer. In some embodiments, at least a portion of the polymer comprises a vinyl alcohol homopolymer or copolymer.

In some embodiments, which can be used in conjunction with any of the above embodiments, the macromer is a hydrophilic macromer or a hydrophobic macromer. In some embodiments, at least a portion of the macromer comprises a vinyl alcohol homopolymer or copolymer.

In some embodiments, which can be used in conjunction with any of the above embodiments, at least a portion of the polymer comprises pendant iodine-containing groups.

In some embodiments, which can be used in conjunction with any of the above embodiments, at least a portion of the macromer comprises pendant iodine-containing groups.

In some embodiments, which can be used in conjunction with any of the above embodiments, the liquid composition further comprises a therapeutic agent.

In some embodiments, the present disclosure pertains to the liquid composition of any of the above embodiments for use as a liquid embolic, a fiducial marker, a tissue-spacing material, or a therapeutic agent depot.

### BRIEF DESCIPTION OF THE DRAWINGS

Fig. 1A is an image showing a string in a tensile test set up. Fig. 1B is an image showing a homogeneous distribution of spherical Ta particles at a microscopic level.
Fig. 2A is a microscopic image of a string without Ta particles (with tensile-test apparatus is shown in inset). Fig. 2B is a microscopic image of a string with spherical Ta particles. Fig. 2C is a microscopic image of a string with nodular Ta particles.
Fig. 3A shows micro-computer tomography (µCT) images of samples containing spherical and nodular Ta particles, along with values reported in greyscale and Hounsfield units. Fig. 3B shows micro-computer tomography (µCT) images of samples containing angular Ta particles, along with values reported in greyscale and Hounsfield units.
Fig. 4A shows X-ray shadowgraphs of the samples shown in Fig. 3A. Fig. 4B shows X-ray shadowgraphs of the samples shown in Fig. 3B.

### DETAILED DESCRIPTION

**In** various aspects, the present disclosure pertains to liquid compositions for medical uses that comprise (a) a polymer, a macromonomer, or a combination thereof and (b) spherical metallic particles. In some embodiments, the spherical metallic particles have a median particle size ranging from 1 to 10 microns.

Spherical metallic particles for use in the present disclosure are spherical metallic particles that are formed from tantalum, tungsten, rhenium, niobium, molybdenum, and their alloys. In particular embodiments the spherical metallic particles are formed from tantalum or a tantalum alloy.

Spherical metallic particles for use in the present disclosure have a generally spherical shape and include spheres, oblate spheroids and prolate spheroids. Median particle size and particle size distribution can be measured by the laser diffraction method and presented as particle size (horizontal axis) versus volume percentage (vertical axis), wherein D10 is the diameter where the portion of the particles with diameters below this value is 10% by volume, D50 is the diameter where portions of particles with diameters smaller and larger than this value are 50% by volume (also referred to herein as the median diameter), and wherein D90 is the diameter where the portion of particles with diameters below this value is 90% by volume. More particularly, laser diffraction measures particle size distributions by measuring the angular variation in intensity of light scattered as a laser beam passes through a dispersed particulate sample. Large particles scatter light at small angles relative to the laser beam and small particles scatter light at large angles. The angular scattering intensity data is then analyzed to calculate the size of the particles responsible for creating the scattering pattern, using the Mie theory of light scattering. The particle size is reported as a volume equivalent sphere diameter.

**In** embodiments, the D10 and/or D90 of the spherical metallic particles may be within 50%, within 40%, within 30%, within 25%, within 20%, within 15%, within 10%, or within 5% of the median diameter (D50).

**In** some embodiments, the spherical metallic particles of the present disclosure can be defined by an average aspect ratio. The average aspect ratio of the spherical metallic particles is defined herein as the ratio of the largest linear dimension of a spherical metallic particle to the smallest linear dimension of the same spherical metallic particle (i.e., tantalum powder) based on measuring randomly 50 particles or 100 particles or measuring randomly about 1 % by weight to about 2 % by weight of a batch of spherical metallic particles. Measurement of the spherical metallic particles is done using Scanning Electron Micrograph (SEM) images. True spherical particles have an aspect ratio of 1.0. In various embodiments, the spherical metallic particles have an average aspect ratio that ranges from 1.0 to 1.25, from 1.0 to 1.15, or from 1.0 to 1.1.

Liquid compositions of the present disclosure include liquid compositions suitable for injection into the body. In various embodiments, such liquid compositions form a solid material *in situ* after injection into the body. For example, some of these liquid compositions rely on polymerization or gel formation to form a solid material *in situ,* while others rely on delivery of a polymeric material in a carrier, such as an organic solvent, which dissipates in the body leaving behind a solid material.

Because the compositions of the present disclosure contain metallic particles, they can have the property of enhanced radiopacity, rendering them visible (or more visible) under X-ray.

In some embodiments, the liquid compositions may contain from 0.1 to 0.9 g/ml of the spherical metallic particles.

The liquid compositions of the present disclosure can be used in a number of applications, including use as liquid embolic compositions, fiducial markers, tissue-spacing materials, and depots which comprise a therapeutic agent and from which the therapeutic agent elutes into the surrounding tissue. The liquid compositions of the present disclosure can also be used to form coatings for the medical devices.

In embodiments wherein the liquid compositions of the present disclosure are injected into the body of a subject, the liquid compositions can be adapted to pass through the particular delivery device employed for the injection, preferably, with manual pressure. The desired viscosity level will typically be dependent on the procedure and the delivery method. For direct injection with a needle and syringe, the amount of pressure required will depend, for example, on the gauge of the needle. Similarly, for injection via catheter, the amount of pressure required will depend, for example, upon the catheter internal diameter. In various embodiments, the viscosity of the liquid compositions will range between 100 mPa·s or less to 20,000 mPa·s or more at 25° C, for example, ranging from 100 mPa·s to 200 mPa·s to 500 mPa·s to 1000 mPa·s to 2000 mPa·s to 5000 mPa·s to 10000 mPa·s to 20000 mPa·s, at 25° C more typically, between 100 to 5000 mPa·s, at 25° C.

The liquid compositions of the present disclosure may be provided in sterile form.

As previously indicated, the presence of spherical metallic particles in the compositions of the present disclosure is advantageous in that the particles will make such compositions more radiopaque during interventional procedures and after implantation in the body.

The presence of spherical metallic particles in the liquid compositions of the present disclosure is also advantageous in that the liquid compositions have improved processability. For example, the particles can be readily dispersed with no need for sonication and have consistent and predictable settling times. Thus, formulation of the liquid compositions at the point of use is improved.

The spherical nature of the particles also allows for reduced particle agglomeration in both the liquid compositions and in the solid compositions formed therefrom. As a result of the reduced agglomeration, the liquid compositions have improved flow properties such that they flow more readily through a given delivery device, reducing injection force and reducing the likelihood of blockage formation within the delivery device. The improved flow also allows the liquid compositions for flow more readily into body cavities in which they are injected and to subsequently fill and conform to the body cavities. In addition, by selecting spherical metallic particles having a uniform size distribution, the particles can pack more tightly and homogeneously after the compositions solidify *in situ,* providing the resulting implant with improved mechanical properties and enhanced radiopacity, as well as a potential reduction in X-ray artefacts arising from the resulting implant (agglomeration can lead to concentrated X-rays and streak artefacts).

As noted above, liquid compositions of the present disclosure include those that solidify *in situ* by various processes when injected into the body, including liquid compositions that precipitate, gel and/or polymerize *in situ.*

In some embodiments, liquid compositions of the present disclosure are precipitating liquid compositions that comprise spherical metallic particles as described above, at least one polymer, and a carrier solvent comprising one or more organic solvents in which the at least one polymer is dissolved. In some embodiments, the one or more organic solvents are water miscible organic solvents. By water miscible is meant that 0.5 ml of the solvent is completely soluble in 1 liter of phosphate buffered saline (137 mM NaCl, 2.7 mM KCl, 10 mM Na₂HPO₄, and 1.8 mM KH₂PO₄) at 20° C. Examples of suitable organic solvents include biocompatible polar aprotic solvents, such as dimethyl sulfoxide, dimethylformamide, N,N'-dimethylpropyleneurea, 1,3-dimethyl-2-imidazolidinone, glycerol, ethyl lactate, N-methyl-2-pyrrolidone and 2-(oxolan-2-yl methoxy)ethanol. The one or more solvents are typically selected from dimethyl sulfoxide and N-methyl-2-pyrrolidone, more typically, dimethyl sulfoxide. In some embodiments, the carrier solvent may comprise water (e.g., up to 80% v/v water) in addition to one or more organic solvents. In other embodiments, the carrier solvent does not contain water. Typically, the precipitating liquid compositions comprise from 2 % wt/wt or less to 50 % wt/wt or more of the at least one polymer dissolved in the solvent, for example, comprising from 2% wt/wt to 5% wt/wt to 10% wt/wt to 25% wt/wt to 50% wt/wt of the at least one polymer dissolved in the solvent, more typically 3 to 35% wt/wt of the at least one polymer dissolved in the solvent.

When the precipitating liquid compositions of the present disclosure are contacted with an environment that stimulates precipitation, for example, physiological fluids, precipitation of the previously dissolved polymer occurs to form a solid *in situ.* During solidification, the carrier solvent diffuses away precipitating liquid compositions. Moreover, diffusion of precipitating species from the body (e.g., non-solvent water or ions) may diffuse into the precipitating liquid compositions. Precipitating liquid compositions offer the advantage that they solidify only when in contact with precipitating stimulus provided by physiological fluids and therefore avoid the problem of solidification within the delivery device, which poses the risk of blockage of the delivery device. In an embolization context, this characteristic allows delivery to be paused during the procedure, for example, in order to prevent flow of material into non-target vessels and allow more control.

In various embodiments, the polymers used in the liquid compositions of the present disclosure, including the precipitating liquid compositions described above, may be selected from various synthetic, natural, or hybrid synthetic-natural hydrophilic or hydrophobic polymers. Examples of polymers include, for example, vinyl alcohol homopolymers and copolymers including poly(vinyl alcohol) (PVA) and ethylene-vinyl alcohol (EVA), alkylene oxide homopolymers and copolymers including poly(ethylene oxide), poly(propylene oxide) and poly(ethylene oxide-co-propylene oxide), vinyl pyrrolidone polymers and copolymers including poly(vinylpyrrolidone) (PVP), allyl alcohol homopolymers and copolymers including poly(allyl alcohol), ethyleneimine homopolymers and copolymers including poly(ethyleneimine), allylamine homopolymers and copolymers including poly(allylamine), vinyl amine homopolymers and copolymers including poly(vinyl amine), oxazolines homopolymers and copolymers including poly(2-alkyl-2-oxazolines) such as poly(2-methyl-2-oxazoline), poly(2-ethyl-2-oxazoline) and poly(2-propyl-2-oxazoline), acrylamide homopolymers and copolymers including poly(N-isopropylacrylamide) (PNIPAM) and polyacrylamide (PAM), acrylate homopolymers and copolymers (including hydroxyalkyl acrylate (e.g., hydroxyethyl acrylate, hydroxypropyl acrylate etc.) homopolymers and copolymers and acrylic acid homopolymers and copolymers), methacrylate homopolymers and copolymers (including hydroxyalkyl methacrylate (e.g., hydroxyethyl methacrylate, hydroxypropyl methacrylate etc.) homopolymers and copolymers and methacrylic acid homopolymers and copolymers), amino acid homopolymers and copolymers, saccharide homopolymers and copolymers, and combinations thereof. In various embodiments, the polymers used in the liquid compositions of the present disclosure are biostable.

In certain particular embodiments, the polymers used in the precipitating liquid compositions of the present disclosure comprise hydroxyethyl methacrylate homopolymers and copolymers.

In certain particular embodiments, the polymers used in the precipitating liquid compositions of the present disclosure comprise vinyl alcohol homopolymers and copolymers, including poly(vinyl alcohol) and poly(ethylene-vinyl alcohol). The vinyl alcohol polymers used in the precipitating liquid compositions described herein (particularly the compositions comprising poly(vinyl alcohol) and poly(ethylene-vinyl alcohol) are typically not crosslinked.

Poly(vinyl alcohol) homopolymers suitable for use in the present disclosure may be of any suitable molecular weight. In some beneficial embodiments, the poly(vinyl alcohol) homopolymers may have a weight average molecular weight ranging from 1 kDa or less to 250 kDa or more, for example, ranging from 1 kDa to 2.5 kDa to 5 kDa to 10 kDa to 25 kDa to 50 kDa to 100 kDa to 250 kDa. In some beneficial embodiments, they may have a weight average molecular weight that is (a) at least 3 kDa and typically at least 5 kDa, and more typically at least 10 kDa and (b) at most 150 kDa, more typically at most 100 kDa, and even more typically at most 75 kDa.

In some embodiments, the liquid composition is a gelling liquid composition that comprises the spherical metallic particles described above, the polymer, and a carrier solvent in which the polymer is dissolved. The carrier solvent may be an aqueous solvent, or a water miscible organic solvent as described elsewhere herein. In some embodiments, the polymer forms a gel in contact with a gelling agent. In some embodiments the gelling agent is a cross linking agent. In some of these embodiments the gelling agent is present naturally in bodily fluids (e.g., divalent and polyvalent cations found in bodily fluids). In some embodiments, the gelling agent is delivered.

In certain particular embodiments, the polymers used in the gelling liquid compositions of the present disclosure comprise or are polysaccharides. In some embodiments the polymer may be, for example, alginate, gellan gum, guar gum, xanthan gum, welan gum, chitosan, hyaluronic acid or starch or mixtures thereof, and particularly alginate or gellan gum.

In some embodiments where the polymer is a polysaccharide, the solvent is an aqueous solvent, such as water. The aqueous solution may comprise a buffering agent, such as a phosphate buffer or other pharmaceutically acceptable buffer.

Polysaccharides may form a gel in contact with a gelling agent. Such agents include divalent and polyvalent cations such as calcium, barium and strontium ions, which may be useful to gel negatively charged polysaccharides such as alginate and gellan gums.

In some of these embodiments, the gelling liquid composition is part of a system that comprises an additional liquid composition comprising the gelling agent. In some embodiments, the additional liquid composition comprising the gelling agent is an aqueous composition.

In some embodiments, the gelling liquid composition is a polymer composition capable of sol-gel phase transition in response to *in vivo* conditions including temperature, ionic strength and/or pH. In some embodiments the polymer composition is capable of sol-gel phase transition in response to both pH and temperature. Such compositions will be a liquid at 20°C, prior to delivery to the body, and a gel at 37°C and pH 7. This composition comprises the spherical metallic particles, the polymer capable of sol-gel phase transition and typically, a carrier solution. In some embodiments the carrier solution is an aqueous carrier solution, in some embodiments the aqueous carrier solution may comprise a pharmaceutically acceptable buffer. In some embodiments, the polymer is a block co-polymer, such as an ABA triblock co polymer. In some embodiments the B block is a pH responsive block and the A block is a temperature sensitive block.

In some embodiments, liquid compositions of the present disclosure are polymerizing liquid compositions that comprise (a) spherical metallic particles as described above and (b) one or more macromonomers, and (c) optionally a carrier solution. The macromonomers polymerize upon contact with a suitable polymerization initiator.

In certain embodiments, the macromonomers of the polymerizing liquid compositions of the present disclosure polymerize upon contact with an initiator that is present in bodily fluids. A fast rate of polymerization may be beneficial in cases where rapid embolization is required such as trauma or gastrointestinal bleeding.

**In** some embodiments, the macromonomers of the polymerizing liquid compositions of the present disclosure polymerize upon contact with a polymerization initiator that is present in an additional liquid composition. In some embodiments, the polymerizing liquid compositions of the present disclosure are part of a system that further comprises the additional liquid composition that comprises the polymerization initiator. The polymerizing liquid composition may be combined with the additional liquid composition before or during delivery to a patient. The polymerizing liquid composition and the additional liquid composition may also be delivered to a patient sequentially, such that the polymerizing liquid composition and the additional liquid composition are combined within the patient *in situ.*

Examples of such polymerization initiators include free-radical initiators. Examples of such macromonomers include free-radical-polymerizable macromers, including polymers that further comprise free-radical-polymerizable groups. Such macromonomers include acylate-terminated macromonomers, methacrylate-terminated macromonomers, and vinyl-terminated macromonomers, among others. Polymers that may be functionalized with polymerizable groups to form macromonomers may be selected from the polymers set forth above. Examples of free radical polymerizable polymers used as, *inter alia,* liquid embolics, can found in WO 01/68720, which discloses free radical polymerizable PVA macromers.

In embodiments where the liquid composition is a polymerizing liquid composition combined with an additional liquid composition that comprises a polymerization initiator before delivery to a patient, or the liquid composition is a gelling liquid composition combined with an additional liquid composition that comprises a gelling agent before delivery to a patient, the components are selected such that the materials solidify over a predictable and predetermined time. In these embodiments, delivery should be carried out within a limited time-frame in order to avoid solidification of the admixed composition in the device, thereby blocking of the device through which the liquid composition is delivered.

In some embodiments where the polymerizing liquid composition is combined with the additional liquid composition that comprises the polymerization initiator during delivery to a patient, or the gelling liquid composition is combined with the additional liquid composition that comprises the gelling agent during delivery to a patient. These liquid compositions and the additional liquid composition can be delivered via a dual-lumen device to ensure the reacting components are kept separate during delivery until mixing at a distal end of the device.

Polymers and macromers used in the liquid compositions of the present disclosure, including those described above, among others, may be provided with a degree of radiopacity by providing the polymers and macromers with pendant iodine-containing groups, for example, by coupling pendant iodine-containing groups along the polymer or macromer backbone. In some embodiments, polymers and macromers in the liquid compositions of the present disclosure may include (a) polymers and macromers, such as those described above among others, that comprise pendant phenyl groups substituted with one or more iodine atoms, (b) polymers and macromers, such as those described above among others, that comprise pendant phenyl groups substituted with one or more iodine atoms and pendant phenyl groups substituted with one or more hydrophilic moieties (e.g., moieties that comprise -OH, -COOH, -SO₃H and/or -OPO₃H₂ groups), and (c) polymers and macromers, such as those described above among others, that comprise pendant phenyl groups that are substituted with both (i) one or more iodine atoms and (ii) one or more one or more hydrophilic moieties. Particular embodiments include iodinated hydroxyethyl methacrylate copolymers such as copolymers of 2,4,6-triidophenol-lactide-lactide-*co*-glycolic acrylate and hydroxyethyl-methacrylate found in precipitating hydrophobic injectable liquid PHIL^{®} (MicroVention, Tustin, CA, USA). Particular embodiments also include iodinated vinyl alcohol polymers and copolymers. Liquid embolic formulations comprising polymers with pendant iodine-containing groups, without spherical metallic particles, are described, for example, in WO02011/110589, WO2020003147, WO2020/003153 and US 2021/0015963.

In certain particular embodiments, the polymer is a polymer comprising a PV A backbone having pendant mono-iodo, di-iodo, tri-iodo or tetra-iodo phenyl groups or combinations of one or more such groups, coupled thereto, particularly through a cyclic acetal group. Such polymers are described in WO2020/003153. In one such polymer the PVA comprises groups of the formula I below

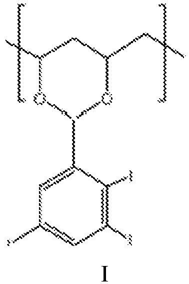

In a further particular embodiment, the polymer comprises a PV A backbone having a first pendant group and a second pendant group, wherein the first pendant group is a mono-iodo, di-iodo, tri-iodo or tetra-iodo phenyl group coupled to the PV A backbone through a cyclic acetal, for example as described above, and wherein the second pendant group is a sulphonated phenyl group coupled to the PV A backbone through a cyclic acetal. An example of such groups is shown in formula II. The sulphonate group may be in the form of a salt such as a sodium salt.

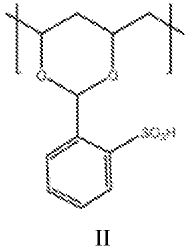

Such polymers are described in WO2021/009734.

In certain particular embodiments, the polymer of the precipitating liquid compositions is an iodinated PV A polymer in which the PV A backbone comprises groups of the formula I and groups of the formula II. In some specific embodiments the solvent is DMSO. In some specific embodiments the precipitating liquid composition comprises 2 to 50 % wt/wt, more typically 3 to 25% wt/wt of the iodinated polymer dissolved in the solvent.

Radiopacity of the polymer or macromer may be varied by adjusting the amount of iodine in the polymer. This may be achieved by varying the number of iodine atoms on the pendant groups or by varying the number of iodine containing pendant groups in the polymer or macromer. Iodine content may be referred to on a dry weight basis for the polymer or macromer. Radiopaque polymers or macromers for use herein may beneficially comprise at least 10%, at least 20%, at least 30%, at least 40%, or at least 50% wt/wt iodine by dry weight of the polymer or macromer. Iodine content may also be referred to on a volumetric basis for the liquid compositions. Iodine content may beneficially comprise at least 10 mg of iodine per cm³, at least 25 mg/cm³ of iodine, at least 50 mg/cm³ of iodine, or at least 100 mg/cm³ of iodine in some embodiments.

In some embodiments, the liquid compositions and solidified compositions described herein may comprise one or more therapeutic agents (e.g., where the liquid compositions are used to form drug depots or drug releasing coatings).

In some embodiments, the liquid compositions and solidified compositions described herein may comprise therapeutic agents that are charged and/or uncharged at physiological pH. The charged therapeutic agents may be electrostatically held within the solidified compositions and subsequently released therefrom by an ion exchange mechanism (e.g., where a polymer and/or macromer in the solidified liquid composition comprises charged groups such as -COO⁻ -SO₃⁻ -OPO₃²⁻, or -NH₃⁺ groups, among others). Charged therapeutic agents electrostatically held in the solidified compositions may elute from the solidified compositions in electrolytic media, such as physiological saline (0.90% w/v NaCl) or in-vivo, e.g., in the blood or tissues, to provide a sustained release of therapeutic agent over several hours, days or even weeks. Uncharged therapeutic agents in the solidified compositions may also elute from the solidified compositions in vivo. This may be particularly advantageous, for example, when rapid elution or a "burst effect" is desired, for example, for rapid therapeutic agent delivery to tissue, or when the low solubility of the therapeutic agent under physiological conditions determines the release profile rather than ionic interaction.

In some embodiments, the liquid compositions and solidified compositions of any of the preceding embodiments may contain from 0.01 mg/ml or less to 10 mg/ml or more of one or more therapeutic agents, for example ranging from 0.01 mg/ml to 0.025 mg/ml to 0.05 mg/ml to 0.10 mg/ml to 0.25 mg/ml to 0.5 mg/ml to 1 mg/ml to 2.5 mg/ml to 5 mg/ml to 10 mg/ml.

Examples of therapeutic agents (which may also be referred to herein as pharmaceutically active ingredients) that can be incorporated into the liquid and solidified compositions of any of the preceding embodiments include small molecule therapeutic agents (defined herein as therapeutic agents having a molecular weight less than 2000 g/mol, typically less than 1500 g/mol, more typically less than 1000 g/mol) and biomolecules (e.g., polypeptides including proteins and protein fragments, such as antibodies and antibody fragments and oligopeptides, as well as polynucleotides and oligonucleotides, including nucleic acids and nucleic acid analogs such as deoxyribonucleic acids, ribonucleic acids, peptide nucleic acids, and fragments thereof).

Examples of therapeutic agents include anti-angiogenic agents, cytotoxic agents, chemotherapeutic agents, checkpoint inhibitors, immune modulatory cytokines, T-cell agonists, and STING (stimulator of interferon genes) agonists, among others.

Examples of therapeutic agents include: checkpoint inhibitors including inhibitors of the binding of PD-1 to PD-L1, inhibitors of the binding of CTLA-4 to CD80 and/or CD86, inhibitors of the binding of TIGIT to CD-112, and inhibitors of the binding of LAG-3 to MHC class II molecules; antibodies or antigen binding fragments thereof that bind to PD-1 (e.g., pembrolizumab, nivolumab domvanalimab, etc.), PD-L1 (e.g., atezolizumab, aveluma, durvalumab, etc.), LAG-3 (e.g., relatlimab, etc.), TIM-3 (e.g., LY3321367, MBG453, TSR-022, etc.), TIGIT (e.g., etigilimab, tiragolumab, vibostolimab, etc.), or CTLA-4 (e.g., ipilimumab tremelimumab, etc.); antibodies or antigen binding fragments thereof that bind to CD3, CD19, CD20, CD22, CD52, CD79B, CD30, CD33, CD38, CD52, CD79B, HER2, EGFR, VEGF, VEGFR2, EPCAM/CD3, GD2, IL-6, RANKL, SLAMF7, CCR4, PDGFRα, Nectin-4 or TROP2; immune modulatory cytokines such as IL-2, IL-12, IL-15, IL-23, interferon gamma (IFN-γ) and gm-CSF (granulocyte macrophage colony stimulating factor); T-cell agonists such as TLR3 agonists (e.g., polyinosinic:polycytidylic acid, double stranded RNAs, etc.), TLR7 agonists (e.g., TMX-202, gardiquimod, imiquimod, etc.), TLR8 agonists (e.g., VTX-2337, etc.), TLR7/8 agonists (e.g., MEDI9197, R848, resiquimod, etc.), TLR9 agonists (e.g., lefitolimod (MGN1703), tilsotolimod, CpG oligodeoxynucleotides (e.g., agatolimod), etc.); and STING agonists such as GSK 532, cyclic dinucleotides (e.g., cyclic guanosine monophosphate-adenosine monophosphate), CRD5500 (LB-061), E7766, ADU-S100, SB11285 MSA2, MK1454, TTI-10001, etc.), among others.

Examples of therapeutic agents further include: camptothecins (such as irinotecan and topotecan) and anthracyclines (such as doxorubicin, daunorubicin, idarubicin and epirubicin), antiangiogenic agents (such as vascular endothelial growth factor receptor (VEGFR) inhibitors, such as axitinib, bortezomib, bosutinib canertinib, dovitinib, dasatinib, erlotinib gefitinib, imatinib, lapatinib, lestaurtinib, masutinib, mubitinib, pazopanib, pazopanib semaxanib, sorafenib, sunitinib, tandutinib, vandetanib, vatalanib and vismodegib), microtubule assembly inhibitors (such as vinblastine, vinorelbine and vincristine), aromatase inhibitors (such as anastrazole), platinum drugs (such as cisplatin, oxaliplatin, carboplatin and miriplatin), nucleoside analogues (such as 5-FU, cytarabine, fludarabine and gemcitabine), paclitaxel, docetaxel, mitomycin, mitoxantrone, bleomycin, pingyangmycin, abiraterone, amifostine, buserelin, degarelix, folinic acid, goserelin, lanreotide, lenalidomide, letrozole, leuprorelin, octreotide, tamoxifen, triptorelin, bendamustine, chlorambucil, dacarbazine, melphalan, procarbazine, temozolomide, rapamycin (and analogues, such as zotarolimus, everolimus, umirolimus and sirolimus), methotrexate, pemetrexed, or raltitrexed.

In some embodiments, the liquid and solidified compositions of any of the preceding embodiments may additionally comprise a therapeutic and/or an imageable radioisotope. Liquid and solidified compositions comprising therapeutic radioisotopes, can be used for example in selective internal radiation therapy (SIRT) or brachytherapy such as in cancer treatment, and may be delivered in any of the manners described elsewhere herein in relation to other embodiments. In one approach, the radioisotope may be bound by ionic interaction or may be covalently attached, such as through a carrier, for example a chelating agent. In some embodiments the radioisotope may be in the form of a particle that is incorporated into the liquid and solidified compositions, wherein the particle comprises the radioisotope. Such particles may be in the form of microspheres, typically having a largest diameter in the range 5um to 500um, particularly less than 100um. The particles may be, for example, polymeric or maybe ceramic. One such ceramic is yttrium aluminosilicate ceramic (see for example US4789501). Further examples of ceramic microspheres are described in WO16082045 and WO05087274). Therapeutic radioisotopes include, but are not limited to, ¹⁷⁷Lu, ⁹⁰Y, ¹³¹I, ⁸⁹Sr, ¹⁵³Sm, ²²³Ra, ²²⁴Ra, ²¹¹At, ²²⁵Ac, ²²⁷Th, ²¹²Bi, ²¹³Bi, and/or ²¹²Pb. In some embodiments, the therapeutic radioisotope is one or more of ¹⁷⁷Lu, ⁹⁰Y, ¹³¹I, ⁸⁹Sr, ¹⁵³Sm, and/or ²²³Ra. In some embodiments, the therapeutic radioisotope is ⁹⁰Y. Imageable radioisotopes include, but are not limited to ^{99m}Tc, ²⁰¹Th , ⁵¹Cr, ⁶⁷Ga, ⁶⁸Ga, ¹¹¹In, ⁶⁴Cu, ⁸⁹Zr, ⁵⁹Fe, ⁴²K, ⁸²Rb, ²⁴Na, ⁴⁵Ti, ⁴⁴Sc, ⁵¹Cr and ¹⁷⁷Lu. In some embodiments the imageable isotope is ^{99m}Tc, ⁶⁷Ga, ⁶⁸Ga, ⁶⁴Cu or ⁸⁹Zr. In some embodiments the imageable isotope is ^{99m}Tc. In some embodiments the imageable isotope is ⁸⁹Zr.

Other embodiments of the present disclosure relate to medical compositions that correspond to, or are formed from, the liquid compositions of any of the preceding embodiments. For example, as previous noted, such liquid compositions can be used to form embolizations, fiducial markers, tissue-spacing materials, therapeutic agent depots, and coatings for medical devices.

Still other embodiments of the present disclosure relate to medical procedures that employ the liquid compositions described herein. For example, in some embodiments, the medical procedures are methods of tissue embolization that comprise delivering the liquid compositions into one or more blood vessels feeding the tissue. Such procedures may be used to treat a variety of conditions including arteriovenous malformations, gastrointestinal bleeding, filling of aneurysms, treatment of solid tumors, particularly hypervascular tumors, such as those of the liver, prostate, kidney, brain, colon, bone and lung, as well as benign hyperplastic conditions such as prostate hyperplasia or uterine fibroids.

In some embodiments, the medical procedures are methods of local or systemic therapeutic agent release that comprise delivering (e.g., by injecting, spraying, etc.) the liquid compositions descried herein to a patient (e.g., onto tissue of the patient, into tissue of the patient, between tissues of the patient, etc.).

In some embodiments, the medical procedures are methods of treatment that comprise delivering (e.g., by injecting, spraying, etc.) the liquid compositions descried herein into or onto a tumor of a patient, wherein the therapeutic agent is released into the tumor.

In some embodiments, the medical procedures are methods of spacing a first tissue from a second tissue that comprise delivering (e.g., injecting, etc.) the liquid compositions descried herein between the first tissue and the second tissue (e.g., between prostate tissue and rectal tissue).

In yet further embodiments, the present disclosure relates to the use of the liquid compositions described herein in the manufacture of a medicament for the treatment of various diseases and conditions, including treatment of arteriovenous malformations, treatment of gastrointestinal bleeding, filling of aneurysms, treatment of solid tumors, particularly hypervascular tumors, such as those of the liver, prostate, kidney, brain, colon, bone and lung, as well as treatment of benign hyperplastic conditions such as prostate hyperplasia or uterine fibroids.

The present disclosure also relates to the use of any of the therapeutic agents described herein in the manufacture of a medicament for the treatment of such diseases and conditions wherein the therapeutic agents is incorporated into a liquid or solidified composition described herein. The present disclosure also relates to the use of any of the therapeutic agents herein in the treatment of such diseases and conditions wherein the therapeutic agents is incorporated into a liquid or solidified composition described herein. The liquid compositions may be particularly used where the liquid composition is delivered by the transcatheter route, by injecting, by implanting by spraying, etc.

### Example 1: Synthesis of iodinated PVA polymer

An iodinated PV A polymer was prepared according to Example 13 of WO2021/009734. Briefly, a dry 600ml HEL Ltd PolyBLOCK^{®} vessel was provided with a nitrogen blanket. Dry DMSO (120ml, 40.2vol) was added with stirring at 500rpm, followed by PVA (146-189kDa. 99% hydrolyzed, 5.0g). The suspension was heated to 50°C until all solids were completely dissolved. 1, 3, 5-triiodobenzaldehyde (TIBA) was then added (10.4g, 0.4eq w.r.t. PVA-1,3-diol units) followed by, 0.05eq of 2-sulfobenzaldehyde sodium salt (Sigma Aldrich UK) (F-SAS). After full dissolution, methanesulfonic acid (11 ml, 3.37vol), in approx. 20mL of cold DMSO was added and stirring continued overnight at 50°C. The pale-yellow solution was cooled to room temperature and precipitated by gradually adding acetonitrile (250mL).

The yellow supernatant was removed by vacuum and the resulting white polymer re-dissolved in DMSO (-100ml) at 50°C and re-precipitated with acetonitrile. Excess solvent was removed by vacuum. The white polymer was suspended in NaOH 0.1N (100mL) for 20min, then blended to achieve a homogeneous suspension. This was neutralized with deionized water (100 x3) until pH=7 after removal of the excess solvent. The obtained white polymer was suspended in acetone (100mL x3) after removing excess water by vacuum and the solid isolated by filtration, using a Büchner funnel. The solid was then dried in a vacuum oven at 28-32°C overnight to give the desired product (white solid, 11-13.0g, -75-80% w/w yield).

### Example 2: General preparation of liquid embolic prototypes

Sample prototypes are prepared by dissolving iodinated PV A prepared according to the above general synthetic protocol, in DMSO. The vials, containing the thick suspension, were then sealed and sonicated until complete dissolution had occurred (typically ca. 4 hours).

### Example 3: General preparation of tantalum suspensions

Tantalum powders were suspended in iodinated PV A preparation by vortexing at 2800rpm for 20mins. Suspensions prepared with spherical Ta powders had a visually more stable distribution of particles compared to suspensions prepared with non-spherical Ta. The following tantalum preparations were obtained:

**Table 1**

| **Tantalum Powder Type** | **Tantalum Powder Identifier** |
|---|---|
| Spherical 1-5µm, Stanford Advanced Materials (Lake Forest, CA USA) Ref. 22016 | Spherical Ta |
| Nodular 1-3µm, Goodfellow (Huntingdon, UK) Ref. TA 006016 | Nodular A |
| Nodular 2-3µm, (Stanford Advanced Materials Lake Forest, CA USA) Ref. ME73-2N8 | Nodular B |
| Angular, 3-10 µm, Global Advanced Metals (Wellesley Hills, MA USA), Ref. GAM PCLS10 | Angular Ta |

### Example 4: Comparison of rheological properties of spherical tantalum, nodular tantalum, and angular tantalum preparations.

Liquid embolic formulations were prepared according to Examples 1 - 3. The formulations comprised either spherical or nodular Ta powder and their rheological properties compared using an Anton Paar MCR 302 rheometer (Anton Paar, St Albans UK) using a cone plate setup with the following settings:
Set up: Cone-Plate
Cone geometry: 60mm (diameter), 1° angle
Cone-plate gap at the measuring position: 0.122mm
Temperature range: 19°C to 40°C
Heating rate: 2°C/min
Shear rate: 50 s⁻¹

The formulations of spherical powder consistently have a lower viscosity than nodular formulations at a given tantalum powder concentration (Table 2).

**Table 2**

| **Tantalum Powder Identifier** | **Tantalum Powder Conc. (% w/w)** | **Viscosity (mPa·s) at 20°C** |
|---|---|---|
| Spherical Ta | 20 | 283 |
| Spherical Ta | 35 | 586 |
| Nodular A | 20 | 320 |
| Nodular A | 35 | 1674 |
| Nodular B | 20 | 322 |
| Nodular B | 35 | 1009 |
| Angular Ta | 20 | 300 |
| Angular Ta | 35 | 643 |

Liquid embolic polymer was prepared using PVA having a molecular weight of 146-189kD comprising 0.4eq of TIBA and 0.075eq of FSAS. The polymer was dissolved in DMSO to obtain a final concentration of 7% w/w (in the final three-component mixture).

### Example 5: Mechanical testing

The polymer of Example 4 was again dissolved in DMSO to obtain a final concentration of 7%w/w. Tantalum samples (spherical, nodular A or angular) were incorporated to achieve a final concentration of 20%w/w or 35%w/w. Specimens for mechanical testing were obtained by extruding the liquid embolic formulation into phosphate-buffered saline (pH 7.0; 37°C; PBS), through a 20µl cylindrical glass micropipette (Drummond, Broomhall, PA, USA) at a constant injection rate of approximately 0.4 ml/min (25 mm/min). This can be done by gluing the pipette into the tip of a Metcal dispenser tip (OK International; Cypress, CA 90630, USA) and delivering the solution using a suitable syringe driving apparatus. The glass pipette tip was maintained in contact with the PBS. The as-formed polymer strings have a cylindrical shape, with regular cross-section of approximately 600µm diameter. Length is dependent on the total injected volume. Samples were stored in deionised water overnight before testing and used as quickly as possible following removal from the storage solution to prevent dehydration.

For each sample, the formed string, was characterized under an optical microscope to verify that it had a regular cylindrical shape. Diameter and length were recorded. The string (wet) was then secured at its extremities on tensile test grips on a universal testing machine (Lloyd (AMETEK) universal testing machine (LF Plus LF1243, PN01471)) and pulled to measure mechanical properties under tensile loads. The following test conditions were used: 500N load cell with a stop set at 300N; initial specimen length: approximately 35 mm; crosshead speed: 60mm/min (constant); specimen cross-section: approximately 600µm.

Tensile test results on strings showed that spherical Ta significantly enhances the mechanical performance of the formed solid embolic, with an increased elongation at break from 100% (nodular A) to 300% (spherical), and an increased Ultimate Tensile Strength from 0.28 MPa (nodular A) to 0.43 MPa (spherical).

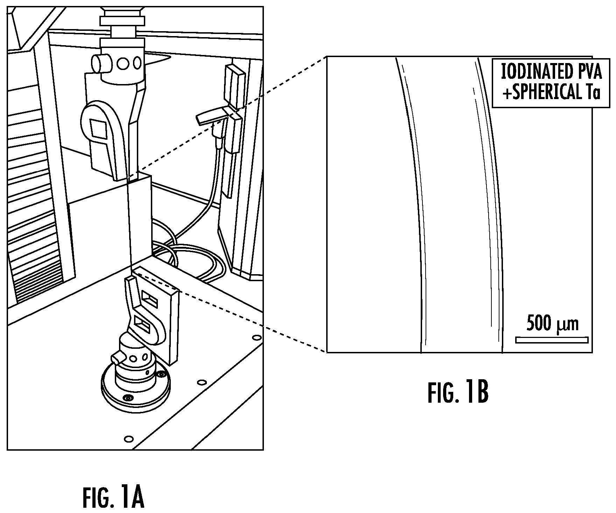

Fig. 1A is an image showing a string in a tensile test set up. Fig. 1B is an image showing a homogeneous distribution of spherical Ta particles at a microscopic level.

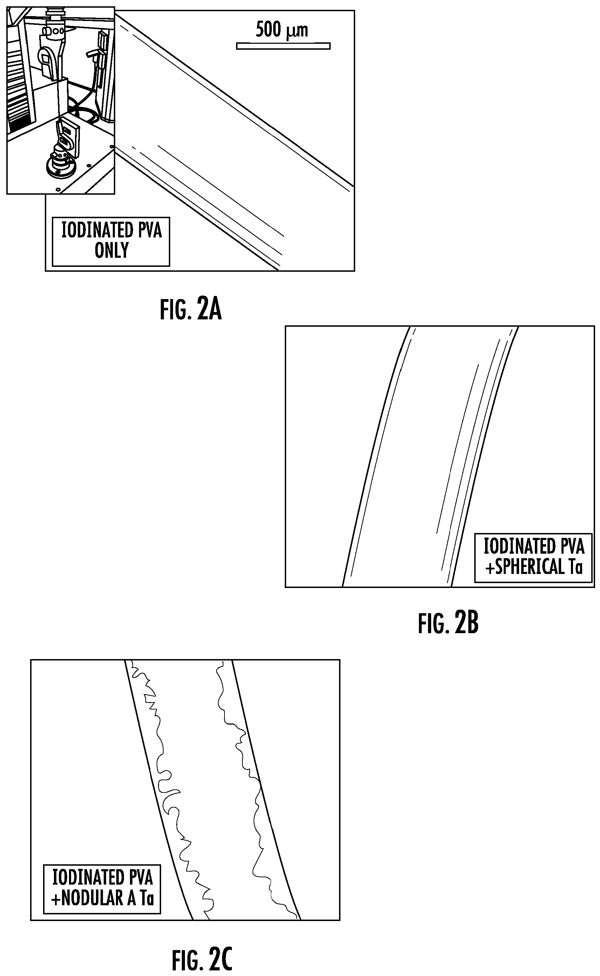

Fig. 2A is a microscopic image of a string without Ta particles (tensile test setup is shown in inset). Fig. 2B is another microscopic image of a string with spherical Ta particles. Fig. 2C is a microscopic image of a string with nodular Ta particles. The spherical Ta preparation was the only one to strongly favour a homogeneous distribution of the Ta particles throughout the formulation and consequently in the formed solid string. Strings obtained from formulations with nodular A Ta showed a very heterogeneous distribution of the particles, with frequent formation of aggregates of the Ta particles.

### Example 6: Imaging

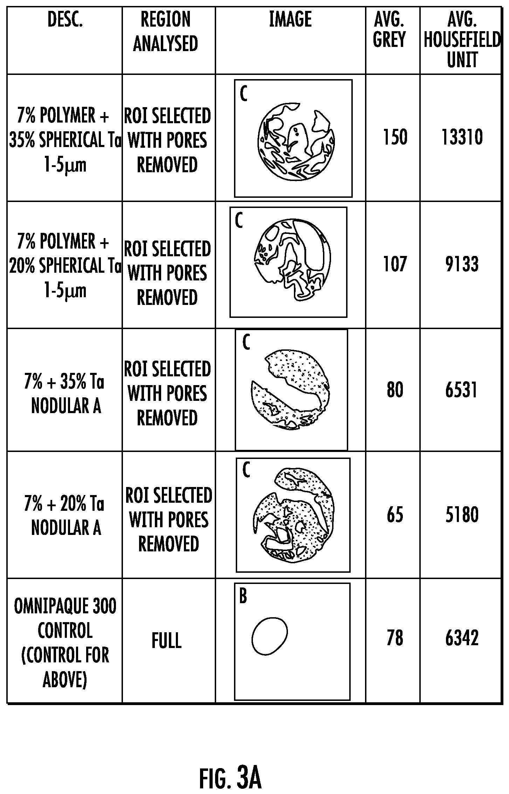

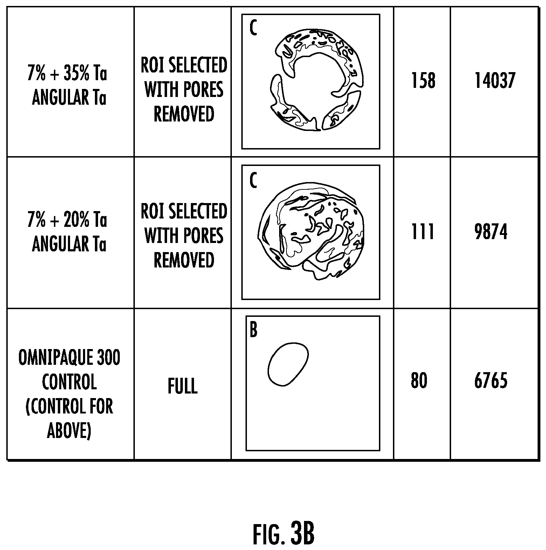

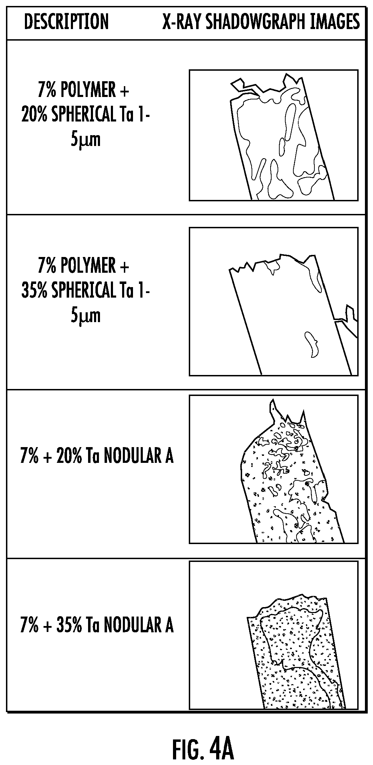

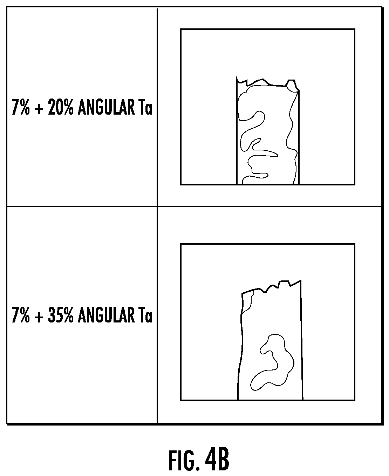

Samples of the polymer threads prepared in Example 5 were suspended in 1% agarose gel (Sigma-Aldrich UK) in Nunc cryotube vials (Sigma-Aldrich V7634, 48 mm × 12.5 mm). These "Bead Phantoms" were imaged using a micro-Computer Tomography (µCT) system (Bruker Skyscan 1172) at the RSSL Laboratories, Reading, Berkshire, UK. The samples were reconstructed using NRecon software and calibrated against a volume of interest (VOI) of a purified water reference. Values were reported in Hounsfield units and greyscale. Typical reconstructed cross sections are shown in Figs. 3A-3B against greyscale and Hounsfield values for the same samples. X-ray shadowgraphs of the same preparations are shown in Figs. 4A-4B.

## Claims

1. A liquid composition for medical use comprising (a) a polymer, a macromonomer, or a combination of the foregoing and (b) spherical metallic particles; wherein at least a portion of the polymer comprises pendant iodine-containing groups and/or wherein at least a portion of the macromonomer comprises pendant iodine-containing groups; and wherein the spherical metallic particles are selected from tantalum, tungsten, rhenium, niobium, molybdenum, and alloys of the foregoing.

2. The liquid composition of claim 1, wherein the spherical metallic particles have one, two or all three of the following characteristics: (a) an average aspect ratio ranging from 1.0 to 1.25, (b) D10 and D90 values that are within 50% of the median diameter, and (c) a median particle size ranging from 1 to 10 microns.

3. The liquid composition of any of claims 1-2, wherein the liquid composition contains from 0.1 to 0.9 g/ml of the spherical metallic particles.

4. The liquid composition of any of claims 1-3, wherein the liquid composition is a sterile, injectable liquid composition that solidifies *in situ* upon injection into a subject.

5. The liquid composition of claim 4, wherein the liquid composition is provided in a syringe barrel.

6. The liquid composition of any of claims 4-5, wherein the liquid composition is a polymerizing liquid composition that comprises the spherical metallic particles, the macromonomer, and optionally a carrier solution, the macromonomer polymerizing upon contact with a polymerization initiator.

7. The liquid composition of any of claims 4-5, wherein the liquid composition is a precipitating liquid composition that comprises the spherical metallic particles, the polymer, and a carrier solvent comprising a water miscible organic solvent in which the polymer is dissolved.

8. The liquid composition of any of claims 4-5, wherein the liquid composition is a gelling liquid composition that comprises the spherical metallic particles, the polymer and a carrier solvent comprising an aqueous or water-miscible organic solvent in which the polymer is dissolved.

9. The liquid composition of any of claims 1-8, wherein at least a portion of the polymer comprises a vinyl alcohol homopolymer or copolymer.

10. The liquid composition of any of claims 1-8, wherein at least a portion of the polymer comprises a polysaccharide.

11. The liquid composition of any of claims 1-6, wherein at least a portion of the macromonomer comprises a vinyl alcohol homopolymer or copolymer.

12. The liquid composition of any of claims 1-11 for use as a liquid embolic, a fiducial marker, a tissue-spacing material, or a therapeutic agent depot.

## Patentansprüche

1. Eine flüssige Zusammensetzung zur medizinischen Verwendung, umfassend (a) ein Polymer, ein Makromonomer oder eine Kombination der vorstehenden und (b) kugelförmige metallische Teilchen; wobei mindestens ein Teil des Polymers seitenständige iodhaltige Gruppen umfasst und/oder wobei mindestens ein Teil des Makromonomers seitenständige iodhaltige Gruppen umfasst; und wobei die kugelförmigen metallischen Teilchen aus Tantal, Wolfram, Rhenium, Niob, Molybdän und Legierungen der vorstehenden ausgewählt sind.

2. Die flüssige Zusammensetzung nach Anspruch 1, wobei die kugelförmigen metallischen Teilchen eine, zwei oder alle drei der folgenden Eigenschaften aufweisen: (a) ein mittleres Seitenverhältnis im Bereich von 1,0 bis 1,25, (b) D10- und D90-Werte, die innerhalb von 50% des Medianwerts des Durchmessers liegen, und (c) einen Medianwert der Teilchengröße im Bereich von 1 bis 10 Mikrometer.

3. Die flüssige Zusammensetzung nach einem der Ansprüche 1-2, wobei die flüssige Zusammensetzung 0,1 bis 0,9 g/ml der kugelförmigen metallischen Teilchen enthält.

4. Die flüssige Zusammensetzung nach einem der Ansprüche 1-3, wobei die flüssige Zusammensetzung eine sterile, injizierbare flüssige Zusammensetzung ist, die sich *in situ* bei Injektion in einen Patienten verfestigt.

5. Die flüssige Zusammensetzung nach Anspruch 4, wobei die flüssige Zusammensetzung in einem Spritzenzylinder bereitgestellt ist.

6. Die flüssige Zusammensetzung nach einem der Ansprüche 4-5, wobei die flüssige Zusammensetzung eine polymerisierende flüssige Zusammensetzung ist, welche die kugelförmigen metallischen Teilchen, das Makromonomer und gegebenenfalls eine Trägerlösung umfasst, wobei das Makromonomer bei Kontakt mit einem Polymerisationsinitiator polymerisiert.

7. Die flüssige Zusammensetzung nach einem der Ansprüche 4-5, wobei die flüssige Zusammensetzung eine sich niederschlagende flüssige Zusammensetzung ist, welche die kugelförmigen metallischen Teilchen, das Polymer und ein Trägerlösungsmittel, welches ein mit Wasser mischbares organisches Lösungsmittel umfasst, in dem das Polymer gelöst ist, umfasst.

8. Die flüssige Zusammensetzung nach einem der Ansprüche 4-5, wobei die flüssige Zusammensetzung eine gelbildende flüssige Zusammensetzung ist, welche die kugelförmigen metallischen Teilchen, das Polymer und ein Trägerlösungsmittel, welches ein wässriges oder mit Wasser mischbares organisches Lösungsmittel umfasst, in dem das Polymer gelöst ist, umfasst.

9. Die flüssige Zusammensetzung nach einem der Ansprüche 1-8, wobei mindestens ein Teil des Polymers ein Vinylalkohol-Homopolymer oder -Copolymer umfasst.

10. Die flüssige Zusammensetzung nach einem der Ansprüche 1-8, wobei mindestens ein Teil des Polymers ein Polysaccharid umfasst.

11. Die flüssige Zusammensetzung nach einem der Ansprüche 1-6, wobei mindestens ein Teil des Makromonomers ein Vinylalkohol-Homopolymer oder -Copolymer umfasst.

12. Die flüssige Zusammensetzung nach einem der Ansprüche 1-11 zur Verwendung als ein flüssiges Emboliemittel, ein Referenzmarker, ein Gewebeabstandsmaterial oder ein Depot für ein Therapeutikum.

## Revendications

1. Composition liquide à usage médical comprenant (a) un polymère, un macromonomère, ou une combinaison des précédents, et (b) des particules métalliques sphériques ; dans laquelle au moins une portion du polymère comprend des groupes iodés pendants et/ou dans laquelle au moins une portion du macromonomère comprend des groupes iodés pendants ; et dans laquelle les particules métalliques sphériques sont choisies parmi le tantale, le tungstène, le rhénium, le niobium, le molybdène, et les alliages des précédents.

2. Composition liquide selon la revendication 1, dans laquelle les particules métalliques sphériques ont une, deux ou toutes les trois des caractéristiques suivantes : (a) un rapport d'aspect moyen situé dans la plage allant de 1,0 à 1,25, (b) des valeurs D10 et D90 situées dans les 50 % du diamètre médian, et (c) une granulométrie médiane située dans la plage allant de 1 à 10 micromètres.

3. Composition liquide selon l'une quelconque des revendications 1 et 2, laquelle composition liquide contient 0,1 à 0,9 g/ml des particules métalliques sphériques.

4. Composition liquide selon l'une quelconque des revendications 1 à 3, laquelle composition liquide est une composition liquide injectable stérile qui solidifie *in situ* après injection dans un sujet.

5. Composition liquide selon la revendication 4, laquelle composition liquide est fournie dans un corps de seringue.

6. Composition liquide selon l'une quelconque des revendications 4 et 5, laquelle composition liquide est une composition liquide polymérisante qui comprend les particules métalliques sphériques, le macromonomère, et éventuellement une solution formant véhicule, le macromonomère polymérisant au contact d'un amorceur de polymérisation.

7. Composition liquide selon l'une quelconque des revendications 4 et 5, laquelle composition liquide est une composition liquide précipitante qui comprend les particules métalliques sphériques, le polymère, et un solvant formant véhicule comprenant un solvant organique miscible avec l'eau dans lequel le polymère est dissous.

8. Composition liquide selon l'une quelconque des revendications 4 et 5, laquelle composition liquide est une composition liquide gélifiante qui comprend les particules métalliques sphériques, le polymère, et un solvant formant véhicule comprenant un solvant organique aqueux ou miscible avec l'eau dans lequel le polymère est dissous.

9. Composition liquide selon l'une quelconque des revendications 1 à 8, dans laquelle au moins une portion du polymère comprend un homopolymère ou copolymère d'alcool vinylique.

10. Composition liquide selon l'une quelconque des revendications 1 à 8, dans laquelle au moins une portion du polymère comprend un polysaccharide.

11. Composition liquide selon l'une quelconque des revendications 1 à 6, dans laquelle au moins une portion du macromonomère comprend un homopolymère ou copolymère d'alcool vinylique.

12. Composition liquide selon l'une quelconque des revendications 1 à 11 pour une utilisation en tant qu'emboligène liquide, marqueur repère, matériau d'espacement tissulaire, ou forme retard d'agent thérapeutique.
